# EUROPEAN PATENT APPLICATION

(11) **EP 4 681 697 A2**
(43) Date of publication of application: **21.01.2026**
(21) Application number: 25199232.7
(22) Date of filing: 24.06.2021
(51) Int. Cl.: A61H 9/00

(54) **VASOCOMPRESSION DEVICES AND METHODS OF DELIVERING VASOCOMPRESSION THERAPY TO A PATIENT UNDERGOING CHEMOTHERAPY TREATMENT**

(30) Priority: 24.06.2020 US 202063043625 P
(62) Divisional of application: 21828395.0
(71) Applicant: Guangzhou Follisave Technology Development Ltd., Guangzhou (CN)
(72) Inventor: BELSON, Amir, Los Altos, CA 94024 (US); BELSON, Ori, Los Altos, CA 94024 (US)
(74) Representative: KIPA AB

(57) **Abstract**

Various devices and methods useful for providing vasocompression to a patient who is undergoing chemotherapy treatment.

## Description

### CROSS REFERENCE TO RELATED APPLICATIONS

This application claims the benefit of U.S. Provisional Patent Application No. 63/043,625, filed June 24, 2020, titled "VASOCOMPRESSION DEVICES AND METHODS OF DELIVERING VASOCOMPRESSION THERAPY TO A PATIENT UNDERGOING CHEMOTHERAPY TREATMENT," which is herein incorporated by reference in its entirety.

### INCORPORATION BY REFERENCE

All publications and patent applications mentioned in this specification are herein incorporated by reference to the same extent as if each individual publication or patent application was specifically and individually indicated to be incorporated by reference.

### FIELD

The various embodiments to which this application relates are directed to devices and methods to mitigate or eliminate chemotherapy induced alopecia (CIA), chemotherapy drug-induced hair loss, hair follicle death and/or anagen effluvium. Additionally or optionally, the devices and methods may be used in conjunction with a medication which aids in these objectives.

### BACKGROUND

Chemotherapeutic agents induce CIA by interfering with the transition between the stages of the hair follicle development, stimulating follicular dystrophy or the induction of premature follicle regression {Paus et al. 1994; Trueb, 2009; Yeager et al. 2011}. Telogen effluvium occurs when a larger proportion of hairs in anagen progress prematurely into the telogen phase; for example, cyclophosphamide causes CIA by this mechanism {Patel et al. 2014}. Anagen effluvium, is the most common kind of CIA, because at any given time up to 90% of scalp hair is in anagen and occurs within days to a few weeks after the administration of cytotoxic agents {Olsen et al. 2011}; it is stimulated by alkylating agents, antimetabolites, vinca alkaloids, topoisomerase inhibitors, anthracyclines and taxanes {Espinosa et al. 2003; Yun et al. 2007}. The hair follicle is particularly sensitive to chemotherapy drugs because, as mentioned above, up to 90% of them are in anagen {Batchelor et al. 2001} with their associated matrix keratinocytes representing the most rapidly dividing cell subset, thus they are especially targeted by cell replication-targeting agents.

Currently, scalp cooling represents the only available approach to prevent chemotherapy induced alopecia and may interfere with CIA via a variety of possible mechanisms. Firstly cooling causes blood vessel vasoconstriction, which has been shown to reduce blood flow in the scalp to 20-40% of the normal rate (Janssen et al. 2007). It has been suggested that this will result in less chemotherapeutic drug being delivered to the hair follicles (Bülow et al. 1985). Another possibility is that the rate of drug diffusion across a plasma membrane may be reduced by cooling and thus lower effective drug doses may enter the cells (Lane et al. 1987). Moreover, as cell division is metabolism-driven, it is possible that this process could be decelerated by cooling as temperature can particularly affect phases such as G1 and S (Watanabe and Okada. 1967), which could be especially important for drugs that target specific phases of the cell cycle, such as microtubule-destructive drugs targeting mitosis. Also a decrease in the metabolic activity of the cells in the hair follicle could cause a more general reduction in the cytotoxicity of chemotherapy drugs (Bülow et al. 1985). In practice, it is likely that a combination of these methods has a role in the success of scalp cooling in reducing CIA.

In any event scalp cooling does not work in all cases and efficacy varies from person to person.

As a result, improvements are still needed in the techniques used to mitigate, substantially eliminate or aid in the treatment of chemotherapy drug-induced hair loss, hair follicle death and/or anagen effluvium.

### SUMMARY OF THE DISCLOSURE

In general, in one embodiment, a head covering for inducing vasocompression in the blood supply of a patient receiving chemotherapy includes a shell having a front edge, a back edge, a top, a left side edge and a right side edge, a liner within the shell shaped to at least partially accommodate the hair of the patient, at least one vasocompression element within the outer shell, and a head covering retention system coupled to the shell.

This and other embodiments can include one or more of the following features. The shell retention system can include a submandibular strap connected to the shell. The shell retention system can include one or more of a rear strap, a front strap or a neck strap connected to the shell. The shell retention system can include one or more adjustment elements. The at least one vasocompression element can be an expandable bladder within the liner. The at least one vasocompression element can be coupled to the shell. The head covering can further include a pressure source in communication with the expandable bladder. The head covering can further include a continuous perimeter connecting the front edge, the back edge, the left side edge and the right side edge. The head covering of the at least one vasocompression element can further include an expandable feature responsive to a pressure source to expand away from the shell. The head covering can further include an expandable layer coupled to the liner. The at least one vasocompression element can be on the expandable layer. The head covering of the at least one vasocompression element can further include one or more raised features. The head covering of the liner within the shell shaped to at least partially accommodate the hair of the patient can further include one or more features on the liner to engage with the hair of the patient. The liner or the at least one vasocompression element can be smooth. The liner or the at least one vasocompression element can include an array of evenly distributed protrusions having straight or circular inflatable structures. The head covering retention system can further include a hook and loop fastener, a buckle, a quick release mechanism or an adjustment feature. The head covering can further include a gas source in communication with the liner or the at least one vasocompression element for inflation of a portion of the liner or a portion of the at least one vasocompression element. The head covering can further include at least one relief valve to prevent over pressurization of an inflatable element of the head covering. The gas source can be a manual pump mechanism, a bulb, an electric pump, or pressurized tank, cartridge or reservoir. The head covering can further include a control system for adjusting the flow of gas from the gas source to maintain a desired pressure within the head covering. The desired pressure within the head covering can be sufficient to produce a vasocompression result while minimizing discomfort or side effects to the patient or harm to surrounding structures. The head covering can further include a cooling gas source in communication with the liner or the at least one vasocompression element wherein a portion of the liner or a portion of the at least one vasocompression element is adapted to provide cooling to an interior portion of the head covering. The head covering can further include one or more pressure sensors within an interior portion of the head covering. The one or more pressure sensors can provide an indication of pressure to the control system. The one or more pressure sensors can be adapted to indicate an amount of pressure applied to a patient undergoing chemotherapy when the patient is wearing the head covering. The head covering can further include one or more sensors to measure skin perfusion (O2 sat) or Doppler blood flow monitor or infra-red pulse oximeter sensor. Sensors may also be used to detect that blood flow in the vasculature receiving vasocompression therapy is stopped or slowed sufficiently to prevent exposure of hair follicles to the chemotherapeutic agent. The head covering can further include a continuous structure from the occiput area to the eyebrows or to the forehead. The head covering can further include a structure or an inflatable structure to compress the eyebrows for a vasocompression effect on the eyebrows. The head covering can further include a timer and a control system adapted to provide an automatic deflation protocol when the timer has elapsed or to cease operation of any active vasocompression element. The head covering can further include a timer and a control system adapted for programmed automatic inflation and deflation of the liner or the one or more vasocompression elements or the activation and deactivation of the one or more vasocompression elements. The liner can be a rigid or semi-elastic layer configured for an exact fit for the patient to fit exactly the shape of the patient's head and hair. The head covering retaining system can include a tightening system. The head covering can further include one or more selective pressure points positioned to compress one or more vessels of the cranial circulatory system of a patient. The head covering can further include a vibrating element coupled to the liner or the at least one vasocompression element. The liner and the at least one vasocompression elements can be sized, shaped, or positioned relative to the head covering based on the hair style of the patient.

In general, in one embodiment, a head covering for inducing vasocompression in the blood supply of a patient receiving chemotherapy includes a cap having a front edge, a back edge, a top, a left side edge and a right side edge, a liner within the cap shaped to at least partially accommodate the hair of the patient, and at least one vasocompression element within the cap.

This and other embodiments can include one or more of the following features. The cap can include a flexible structure, a partially flexible structure, a combination of semi-rigid structure and flexible structure or an adjustable structure sized to remain in place on the head when the at least on vasocompression element is active. The at least one vasocompression element can be an expandable bladder within the cap or the liner. The at least one vasocompression element can be coupled to the cap or the liner. The head covering can further include a pressure source in communication with the expandable bladder or the at least one vasocompression element. The head covering can further include a continuous perimeter connecting the front edge, the back edge, the left side edge and the right side edge. The head covering of the at least one vasocompression element can further include an expandable feature responsive to a pressure source to expand away from the cap. The head covering can further include an expandable layer coupled to the liner wherein the at least one vasocompression element can be on the expandable layer. The head covering of the at least one vasocompression element can further include one or more raised features. The head covering of the liner within the shell shaped to at least partially accommodate the hair of the patient can further include one or more features on the liner to engage with the hair of the patient. The liner or the at least one vasocompression element can be smooth. The liner or the at least one vasocompression element can include an array of evenly distributed protrusions having straight or circular inflatable structures. The head covering can further include a gas source in communication with the liner or the at least one vasocompression element for inflation of a portion of the liner or a portion of the at least one vasocompression element. The head covering can further include at least one relief valve to prevent over pressurization of an inflatable element of the head covering. The gas source can be a manual pump mechanism, a bulb, an electric pump, or pressurized tank, cartridge or reservoir. The head covering can further include a control system for adjusting the flow of gas from the gas source to maintain a desired pressure within the head covering. The desired pressure within the head covering can be sufficient to produce a vasocompression result while minimizing discomfort or side effects to the patient or harm to surrounding structures. The head covering can further include a cooling gas source in communication with the liner or the at least one vasocompression element wherein a portion of the liner or a portion of the at least one vasocompression element is adapted to provide cooling to an interior portion of the head covering. The head covering can further include one or more pressure sensors within an interior portion of the head covering. The one or more pressure sensors can provide an indication of pressure to the control system. The one or more pressure sensors can be adapted to indicate an amount of pressure applied to a patient undergoing chemotherapy when the patient is wearing the head covering. The head covering can further include one or more sensors to measure skin perfusion (O2 sat) or Doppler blood flow monitor or infra-red pulse oximeter sensor. The head covering can further include a continuous structure from the occiput area to the eyebrows or to the forehead. The head covering can further include a structure or an inflatable structure to compress the eyebrows for a vasocompression effect on the eyebrows. The head covering can further include a timer and a control system adapted to provide an automatic deflation protocol when the timer has elapsed or to cease operation of any active vasocompression element. The head covering can further include a timer and a control system adapted for programmed automatic inflation and deflation of the liner or the activation and deactivation of the one or more vasocompression elements. The liner can be a rigid or semi-elastic layer configured for an exact fit for the patient to fit exactly the shape of the patient's head and hair. The head covering retaining system can include a tightening system. The head covering can further include one or more selective pressure points positioned to compress one or more vessels of the cranial circulatory system of a patient. The head covering can further include a vibrating element coupled to the liner or the at least one vasocompression element. The liner and the at least one vasocompression elements can be sized, shaped, or positioned relative to the head covering based on the hair style of the patient.

In general, in one embodiment, a method of inducing vasocompression in a cranial circulatory system of a patient receiving chemotherapy includes: (1) placing a head covering about the head of the patient; (2) at least partially inducing vasocompression in a portion of the cranial circulatory system of the patient using a vasocompression element of the head covering; (3) Initiating a chemotherapy session with the patient; (4) concluding the chemotherapy session with the patient; and (5) ceasing the step of at least partially inducing vasocompression in a portion of the cranial circulatory system of the patient when a period of time has elapsed after the step of concluding the chemotherapy session sufficient to partially mitigate an effect of chemotherapy induced alopecia in the patient.

This and other embodiments can include one or more of the following features. The method can further include engaging a head covering retention system to immobilize the head covering relative to the head of the patient. The period of time that has elapsed after the step of concluding the chemotherapy session can be up to 24 hours. The period of time that has elapsed after the step of concluding the chemotherapy session can be at least one hour. The period of time that has elapsed after the step of concluding the chemotherapy session is can be least 48 hours. The vasocompression element of the head covering can be a liner wherein a step of expanding the liner or a step of contracting the liner is performed during the step of at least partially inducing vasocompression in a portion of the cranial circulatory system of the patient. There can be an increase in the amount of vasocompression during the step of expanding the liner corresponding to an increase of the force of the liner against the scalp of the patient. There can be a decrease in the amount of vasoconstriction during the step of contracting the liner corresponding to a decrease of the force of the liner against the scalp of the patient. The vasocompression element of the head covering can be a liner having one or more features wherein a step of expanding the liner or a step of contracting the liner is performed during the step of at least partially inducing vasocompression in a portion of the cranial circulatory system of the patient. There can be an increase in the amount of vasocompression during the step of expanding the liner corresponding to an increase of the force of the one or more features on the liner against the scalp of the patient. There can be a decrease in the amount of vasocompression during the step of contracting the liner corresponding to a decrease of the force of the one or more features on the liner against the scalp of the patient. The vasocompression element of the head covering can be an inflatable bladder wherein the degree of vasocompression during the step of at least partially inducing vasocompression in a portion of the cranial circulatory system of the patient corresponds at least in part to the amount of pressure within the inflatable bladder. The vasocompression element of the head covering can be an inflatable bladder wherein the degree of vasocompression during the step of at least partially inducing vasocompression in a portion of the cranial circulatory system of the patient is increased or decreased by raising or lowering the pressure in the inflatable bladder against the skull. The vasocompression element of the head covering can be one or more features arranged on an interior surface of the head covering which are urged into contact with a portion of the patient's scalp having hair follicles during the step of at least partially inducing vasocompression in a portion of the cranial circulatory system of the patient. The vasocompression element of the head covering can be one or more features arranged on an interior surface of the head covering which correspond to an arrangement of the patient's hair, wherein the one of more features act on the arrangement of the patient's hair to further the performance of the step of least partially inducing vasocompression in a portion of the cranial circulatory system of the patient. During the at least partially inducing vasocompression step, the vasocompression element of the head covering can compress a portion of the scalp containing a plurality of hair follicles against a portion of the cranium to at least partially vasocompress the blood supply to the plurality of hair follicles. The portion of the cranium can be one or more of a superior temporal line, an inferior temporal line, a parietal bone, a squamous suture, a temporal bone, a lambdoid suture, an occipital bone, a mastoid process, a sphenoid, a portion in proximity to the supraorbital foramen, a frontal bone, a coronal suture. During the at least partially inducing vasocompression step, the vasocompression element of the head covering can be positioned to at least partially vasocompress an artery or a vein of the cranial circulatory system of the patient by compressing the artery or the vein against a portion of the cranium. The artery or the vein of the cranial circulatory system can be a branch of an external carotid artery or a branch of the internal carotid artery. The artery or the vein of the cranial circulatory system can be an ophthalmic artery. The artery or the vein of the cranial circulatory system can be a superficial temporal artery. The artery or the vein of the cranial circulatory system can be a posterior auricular artery. The artery or the vein of the cranial circulatory system can be an occipital artery. The artery or the vein of the cranial circulatory system can be a supraorbital artery or a supratrochlear artery. The portion of the cranium can be one or more of a superior temporal line, an inferior temporal line, a parietal bone, a squamous suture, a temporal bone, a lambdoid suture, an occipital bone, a mastoid process, a sphenoid, a portion in proximity to the supraorbital foramen, a frontal bone, a coronal suture. The step of at least partially inducing vasocompression in a portion of the cranial circulatory system can result from compression of a portion of the scalp containing a blood vessel of the cranial circulatory system of the patient against a portion of the periosteum of the patient. The step of at least partially inducing vasocompression in a portion of the cranial circulatory system can be produced by interaction between the vasocompression element of the head covering and a blood vessel of the cranial circulatory system of the patient. The method of the step of at least partially inducing vasocompression in a portion of the cranial circulatory system can further include compressing a portion of the scalp against a portion of the periosteum to induce vasocompression in a portion of a vascular bed of a hair follicle. The vasocompression element of the head covering can be positioned with respect to the patient's head wherein during the engaging step the vasocompression element is positioned in proximity a specific vein or a specific artery of the cranial circulatory system of the patient to at least partially vasocompress the specific vein or the specific artery. The specific vein or the specific artery can be any of the blood vessels mentioned above. The method can further include delivery of a vasoconstrictor medication to the patient before, during or after use of any methods or devices described herein. The vasoconstrictor medication can be one of an alpha-adrenoceptor agonist, a vasopressin analog, an epinephrine, a norepinephrine, a phenylephrine, a dopamine, a dobutamine, a migraine medication, a headache medication, a serotonin 5-hydroxytryptamine agonist, and a triptans. The method or device as in any of the above, wherein the patient can be receiving chemotherapy by an injection, an infusion, a targeted therapy, a hormone therapy or an immunotherapy. The patient can be receiving chemotherapy by an intravenous method, an intrathecal method, an intra-arterial method, an intracavitary method, an intramuscular method, an intralesional method, or an intravesical method. The patient can be receiving chemotherapy in a home, a health care provider office, a clinic, an outpatient infusion center, a hospital infusion center, or a hospital in-patient room.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIG. 1A is a perspective view of a cancer patient at the beginning of a chemotherapy treatment to treat a tumor or malignant lesion in a breast. This view includes a bag containing a chemotherapeutic agent and the amount in the bag at the initiation of the chemotherapeutic treatment session.
FIG. 1B is an enlarged view of the tumor or malignant lesion in the breast in the patient of FIG.1A.
FIG. 2A is a view of the bag in FIG. 1A showing the amount delivered and remaining of the chemotherapeutic agent.
FIG. 2B is a graph showing an expected concentration curve of the chemotherapeutic agent in the patient's blood stream as a function of Drug Concentration (mg/L) and Time (min). The triangle marks the estimated concentration based on the amount of chemotherapy agent delivered as indicated by the level of fluid visible in the bag.
FIG. 3A is a view of the patient and the bag in FIG. 1A showing the amount delivered and remaining of the chemotherapeutic agent as indicated by the decreased level of fluid in the bag.
FIG. 3B is the graph of FIG. 2B showing an expected concentration curve of the chemotherapeutic agent in the patient's blood stream as a function of Drug Concentration (mg/L) and Time (min) for the status of the patient in FIG. 3A. The triangle marks the estimated concentration based on the amount of chemotherapy agent delivered as indicated by the decreased level of fluid visible in the bag which is nearly at the peak or maximum drug concentration.
FIG. 4 is a view of the state of a process of chemotherapy-induced necrosis in the targeted tumor or malignant lesion of FIG. 1B.
FIG. 5A is a view of the patient and the bag in FIG. 3A showing the amount delivered and remaining of the chemotherapeutic agent as indicated by the decreased level of fluid in the bag. A Drug Concentration (mg/L) and Time (min) curve for the patient in FIG. 5A would be at, near or beyond the highest level.
FIG. 5B is a view of the advancing state of the process of chemotherapy-induced necrosis in the targeted tumor or malignant lesion of FIGs. 1B and 4.
FIG. 5C is a cross section of the scalp and a hair follicle including the vascular bed before any chemotherapeutic agent reaches the vasculature of the skull, scalp or vascular bed of the hair.
FIG. 5D is a cross section of the scalp and a hair follicle vascular bed of FIG. 5C showing the infiltration and circulation of the chemotherapeutic agent.
FIG. 6A is a view of the final state of chemotherapy-induced necrosis in the targeted tumor or malignant lesion of FIGs. 1B, 4 and 5B.
FIG. 6B is a view of the final state of wide spread chemotherapy-induced necrosis in the follicles of the patient which began in FIG. 5D.
FIG. 6C is a side view of the state of wide spread hair loss in the patient of FIGs. 1A, 3A, and 5A produced by one or a combination of chemotherapy-induced alopecia, hair loss, hair follicle death and/or anagen effluvium.
FIG. 7A is a perspective view of a pulled back portion of the skin and scalp to reveal the layers of tissue over a bony portion of a skull.
FIG. 7B is a cross section of view of the pulled back portion of the skin and scalp of FIG. 7A.
FIG. 7C is a cross section of view of the indicated area of the skin and scalp of FIG. 7B.
FIG. 8 is a cross section of a healthy scalp and a hair follicle including the vascular bed.
FIG. 9 is a perspective view of a head covering for inducing vasocompression in the blood supply of a patient receiving chemotherapy. This view also shows a shell and interior liner which is connected to an external pump or controller for one or more vasocompression elements in the head covering. A submandibular strap connected to the shell is also visible in this view. The head covering is configured to deliver vasocompression to the scalp and eyebrows.

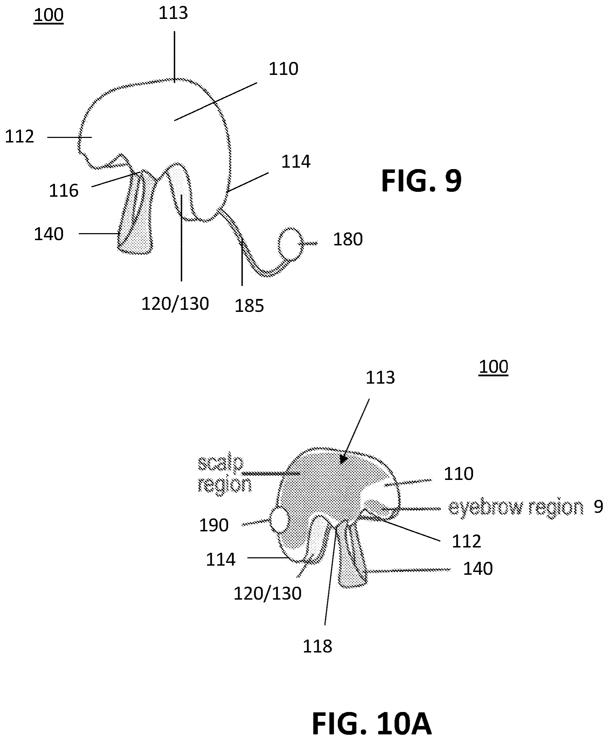

FIG. 10A is a perspective view and partial interior view of a head covering for inducing vasocompression in the blood supply of a patient receiving chemotherapy. This view also shows a shell and interior liner which is connected to a shell mounted pump or controller for one or more vasocompression elements in the head covering. A submandibular strap connected to the shell is also visible in this view. The vasocompression element illustrated is an inwardly expanding manifold where inwardly is toward scalp - away from shell. The head covering is configured to deliver vasocompression to the scalp and eyebrows.
FIG. 10B is a cross section view of a portion of a liner having a vasocompression element with one or more raised features. The raised features have a rounded shape facing the patient who is wearing the head covering.
FIG. 10C is a cross section view of a portion of a liner having a vasocompression element with one or more raised features. The raised features have a rectangular pyramid shape with a flat top section facing the patient who is wearing the head covering.
FIG. 11A is a side view of a patient's head who is wearing a vasocompression element used to deliver vasocompression in the blood supply of the patient while receiving chemotherapy. This view also shows an inflatable liner which is connected to a pump or controller (not visible in this view). The inflatable liner vasocompression element is configured to deliver vasocompression to the scalp and eyebrows.
FIG. 11B is a side view of the patient in FIG. 11A showing the shell and an under the chin strap of the head covering for inducing vasocompression in the blood supply of a patient receiving chemotherapy using the vasocompression element of FIG. 11A to deliver vasocompression to the scalp and eyebrows.
FIG. 12A is a side view of a patient's head who is wearing a head covering including at least one vasocompression element used to deliver vasocompression. This is the state of the patient prior to or shortly after initiation of the patient receiving chemotherapy. A submandibular strap connected to a shell of the head covering is also visible in this view. The head covering is configured to deliver vasocompression to the scalp and eyebrows of the patient.
FIG. 12B is a cross section of a portion of the hair, scalp and hair follicles of the patient in FIG. 12A. This view shows the hair pressed against the scalp when the head covering in donned (FIG. 12A) and prior to the delivery of vasocompression.
FIG. 12C is a cross section of a portion of the hair, scalp and hair follicles of the patient in FIG. 12B. This view shows the expansion of one or more vasocompression elements in the head covering causing vasocompression within the vascular bed of the follicle.
FIG. 12D is an exterior view of the patient undergoing chemotherapy and vasocompression with arrows indicating that vasocompression may be applied to numerous portions of the scalp including the eyebrows.
FIG. 13A is a view of the patient of FIGs. 12A-12D receiving vasocompression from the head covering during a chemotherapy session which is nearly completed as indicated by the nearly empty bag of chemotherapeutic agent.
FIG. 13B is the graph showing an expected concentration curve of the chemotherapeutic agent in the patient's blood stream as a function of Drug Concentration (mg/L) and Time (min) for the status of the patient in FIG. 13A. The triangle marks the estimated concentration based on the amount of chemotherapy agent delivered as indicated by the decreased level of fluid visible in the bag of FIG. 13A. The arrow indicates that the drug concentration amount is nearly at or just beyond the peak or maximum drug concentration.
FIG. 13C is a view of the advancing state of the process of chemotherapy-induced necrosis in the targeted tumor or malignant lesion of the patient of FIGs. 12A, 12D and 13A who has been receiving vasocompression during the delivery of the chemotherapeutic agent.
FIG. 13D is a cross section of the head covering, scalp and a hair follicle of the patient of FIGs. 12A, 12D and 13A who has been receiving vasocompression during the delivery of the chemotherapeutic agent (FIG. 13B). This view shows that the vascular bed is completely free or substantially free of any chemotherapeutic agent or that the level of chemotherapeutic agent that reaches the vascular bed is maintained below a level to harm the follicles.
FIG. 14 is a side view of the patient of FIG. 13A at a time period after the delivery of chemotherapy with vasocompression. The patient has maintained hair on both the head and the eyebrows as a result of the beneficial effects of vasocompression.
FIG. 15 is a flow chart of a representative method for providing vasocompression to a patient undergoing a chemotherapy treatment.

### DETAILED DESCRIPTION

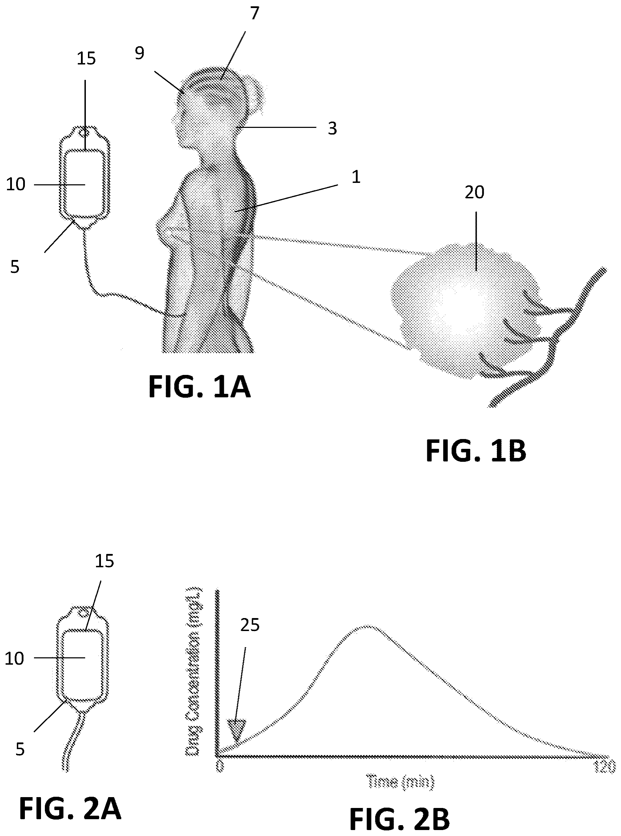

FIG. 1A is a perspective view of a cancer patient 1 at the beginning of a chemotherapy treatment to treat a tumor or malignant lesion 20 in a breast. This view includes an IV bag 5 containing a chemotherapeutic agent 10 and the amount in the bag 15 at the initiation of the chemotherapeutic treatment session.

FIG. 1B is an enlarged view of the tumor or malignant lesion 20 in the breast in the patient 1 of FIG.1A.

FIG. 2A is a view of the IV bag 5 in FIG. 1A showing the amount delivered and remaining 15 of the chemotherapeutic agent 10.

FIG. 2B is a graph showing an expected concentration curve of the chemotherapeutic agent 10 in the patient's blood stream as a function of Drug Concentration (mg/L) and Time (min). The triangle 25 marks the estimated concentration based on the amount of chemotherapy agent 10 delivered as indicated by the level of fluid 15 visible in the bag 5.

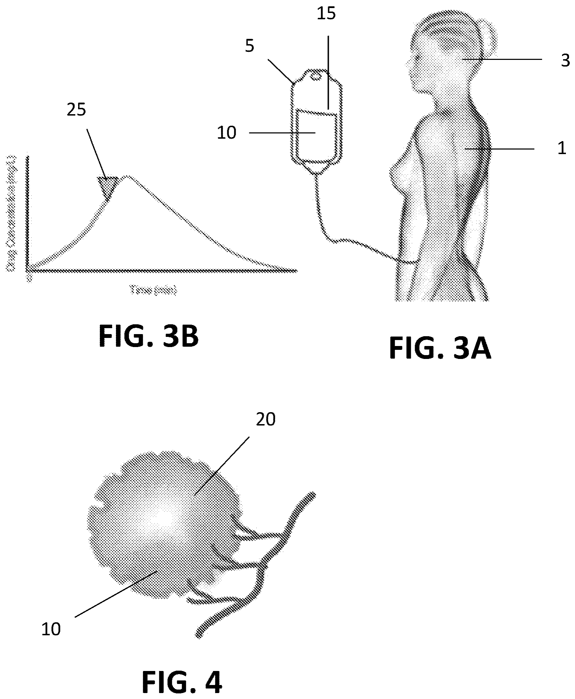

FIG. 3A is a view of the patient and the IV bag 5 in FIG. 1A showing the amount delivered 15 and remaining of the chemotherapeutic agent 10 as indicated by the decreased level of fluid in the bag.

FIG. 3B is the graph of FIG. 2B showing an expected concentration curve of the chemotherapeutic agent 10 in the patient's blood stream as a function of Drug Concentration (mg/L) and Time (min) for the status of the patient in FIG. 3A. The triangle 25 marks the estimated concentration based on the amount of chemotherapy agent delivered as indicated by the decreased level of fluid visible in the bag which is nearly at the peak or maximum drug concentration.

FIG. 4 is a view of the state of a process of chemotherapy-induced necrosis in the targeted tumor or malignant lesion of FIG. 1B.

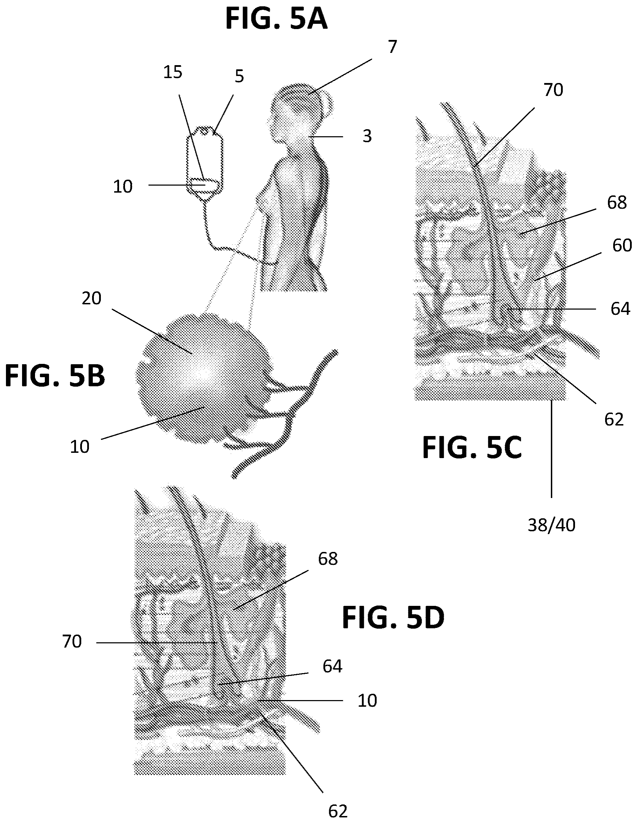

FIG. 5A is a view of the patient and the bag in FIG. 3A showing the amount delivered 15 and remaining of the chemotherapeutic agent 10 as indicated by the decreased level of fluid 15 in the IV bag 5. A Drug Concentration (mg/L) and Time (min) curve for the patient in FIG. 5A would be at, near or beyond the highest level. (See for example the peak level in FIG. 3B)

FIG. 5B is a view of the advancing state of the process of chemotherapy-induced necrosis in the targeted tumor or malignant lesion 20 of FIGs. 1B and 4. A large amount of the chemotherapeutic agent 10 has been delivered to and absorbed by the tumor or lesion 20.

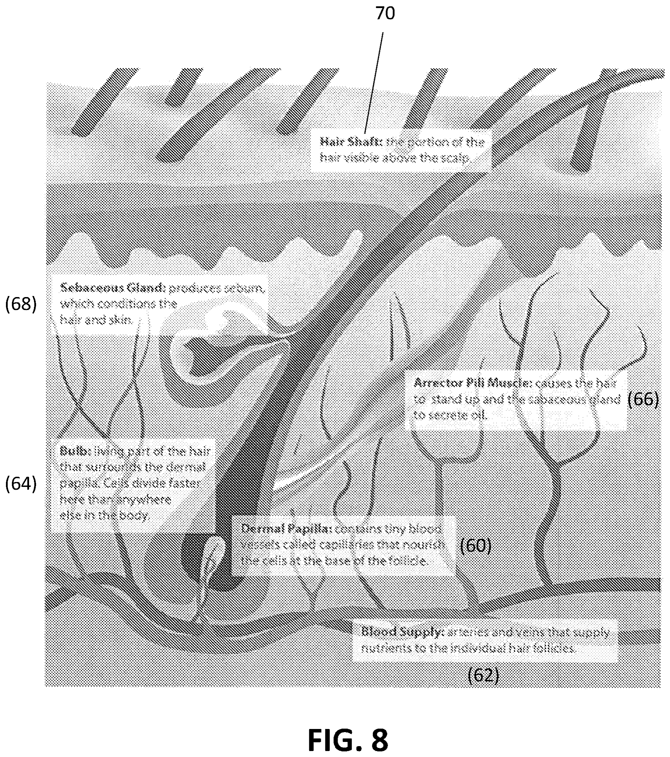

FIG. 5C is a cross section of the scalp and a hair follicle similar to FIG 8. In this view, the vascular bed is shown before any chemotherapeutic agent 10 reaches the vasculature of the skull, scalp or vascular bed of the hair.

FIG. 5D is a cross section of the scalp and a hair follicle vascular bed of FIG. 5C showing the infiltration and circulation of the chemotherapeutic agent 10 within the vasculature 62.

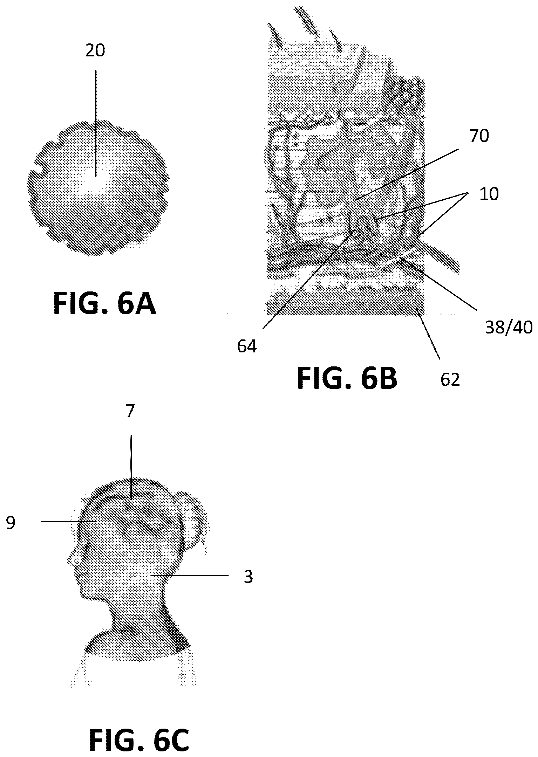

FIG. 6A is a view of the final state of chemotherapy-induced necrosis in the targeted tumor or malignant lesion 20 of FIGs. 1B, 4 and 5B.

FIG. 6B is a view of the final state of wide spread chemotherapy-induced necrosis in the follicles of the patient which began in FIG. 5D. The vasculature 62 contains the chemotherapeutic agent 10 which has been delivered to and destroyed the bulb 64 and associated hair shaft 70. Introduction of the chemotherapeutic agent into the vascular bed of the scalp and eye brows produces in the patient one or more chemotherapy induced alopecia (CIA), chemotherapy drug-induced hair loss, hair follicle death and/or anagen effluvium.

FIG. 6C is a side view of the state of wide spread hair loss in the patient of FIGs. 1A, 3A, and 5A produced by one or a combination of chemotherapy-induced alopecia, hair loss, hair follicle death and/or anagen effluvium. The amount of hair 7 on the head 3 and eyebrows is less than the amounts in the prior views of FIG. 1A, 3A and 5A.

The deleterious consequences described above may be mitigated or avoided to a large extend using an embodiment of a device and method described herein that impede or prevent chemotherapeutic agents from reaching the hair follicles. As used herein, vasocompression refers to a force applied to the head of a patient sufficient to overcome filling pressure or systolic pressure in the arterial supply to the scalp. Put another way, vasocompression achieved by the various embodiments and methods described herein block off the arterial blood supply to the scalp so that chemotherapeutic agents in the blood stream during chemotherapy are prevented from reaching the hair follicle blood supply.

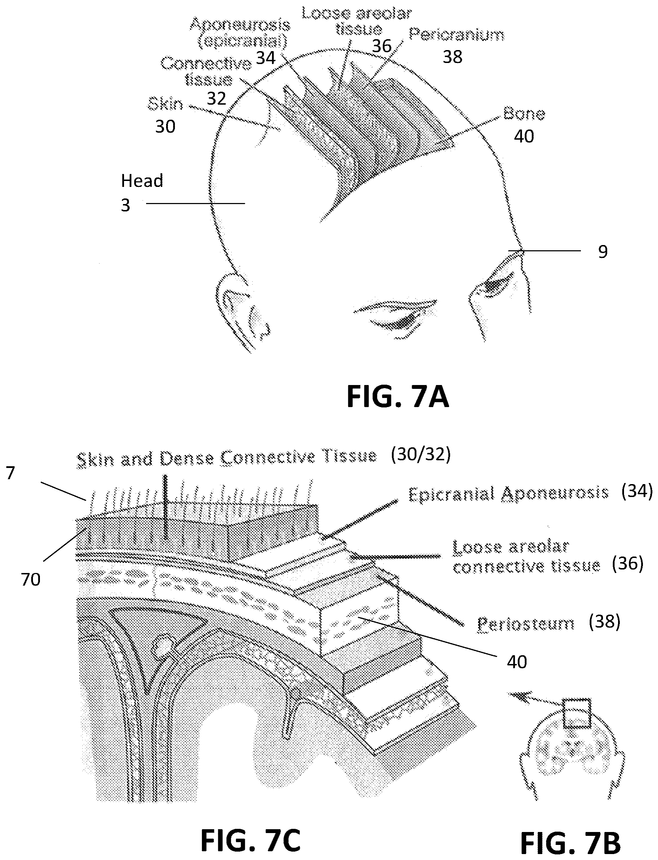

FIG. 7A is a perspective view of a head 3 of a patient 1 with pulled back portion of the skin 30 and scalp to reveal the layers of tissue over a bony portion of a skull. Beneath the skin 30 is shown the connective tissue 32, aponeurosis or epicranial layer 34 and the loose areolar tissue 36. Below the loose areolar layer 36 is the pericranium 38 and then bone 40.

FIG. 7B is a cross section of view of the pulled back portion of the skin and scalp of FIG. 7A. FIG. 7C is a cross section of view of the indicated area of the skin and scalp of FIG. 7B. This view illustrates the relationship of the skin and dense connective tissue (30/32) to the epicranial aponeurosis 34, the loose areolar connective tissue 36 and the periosteum 38 and bone 40.

FIG. 8 is a cross section of a healthy scalp and a hair follicle including the vascular bed. The blood supply 62 includes arteries and veins that supply nutrients to the individual hair follicles. The hair shaft 70 and bulb 64 are shown in relation to the dermal papilla 60 and blood supply 62. The sebaceous gland 68 and arrector pili muscle 66 are also shown in this view.

FIG. 9 is a perspective left side view of an embodiment of a head covering 100 for inducing vasocompression in the blood supply of a patient 1 receiving chemotherapy. This view also shows a shell 110 along with a front edge 112, a top 113, a left side edge 116 and a back edge 114. Also shown in this view is portion of the head covering interior with a portion of the liner 120 and one or more vasocompression elements 130 visible. The one or more vasocompression elements 130 are connected to an external pump or controller 180 using a suitable connector 185. The pump or controller 180 is used to execute the desired vasocompression therapy to the patient using the one or more vasocompression elements 130 in the head covering 100. A submandibular strap connected to the shell is also visible in this view. The head covering 100 has a shell 110 adapted and configured to deliver vasocompression to the scalp with a front edge 112 selected to provide vasocompression to the eyebrows. In one aspect, one or more vasocompressive elements 130 may be placed continuously along the liner or interior of the shell to treat the scalp/hair 7 and eyebrows 9 and the space on the forehead between the hair line and the eyebrows. Additionally or optionally, separate vasocompression elements 130 are used for the eyebrows and the hair with no elements 130 in the forehead region between the hair line and the eyebrows.

FIG. 10A is a perspective right side view and partial interior view of an embodiment of a head covering 100 for inducing vasocompression in the blood supply of a patient 1 receiving chemotherapy. This view also shows a shell 110 with a top 113, a front edge 112, a back edge 114 and a right-side edge 118. The shell 110 is illustrated as transparent to show the location of the vasocompression elements 130 aligned to delivery vasocompression therapy to the hair/scalp 7 and eyebrows 9 of the patient 1. Also shown in this view is an interior portion of the liner 120 adjacent to the left side edge 116. In this embodiment of the head covering, the pump or controller 190 is mounted directly onto the head covering 100 - such as to a portion of the shell 110. The pump or controller 190 - like the pump or controller 180 - is appropriately connected to the one or more vasocompression elements 130 in the head covering 100. The mounted pump or controller 190 is used to execute the desired vasocompression therapy to the patient using the one or more vasocompression elements 130 in the head covering 100. A submandibular strap 145 connected to the shell 110 is also visible in this view. The vasocompression element 130 illustrated is an inwardly expanding manifold where inwardly is toward scalp - away from shell 110. The head covering 100 is configured to deliver vasocompression to the scalp and eyebrows 9 as can be seen by the size, orientation and location of the vasocompression element 130 positioned adjacent to the scalp and another separate or spaced apart vasocompression element 130 positioned for engagement with the eyebrows 9.

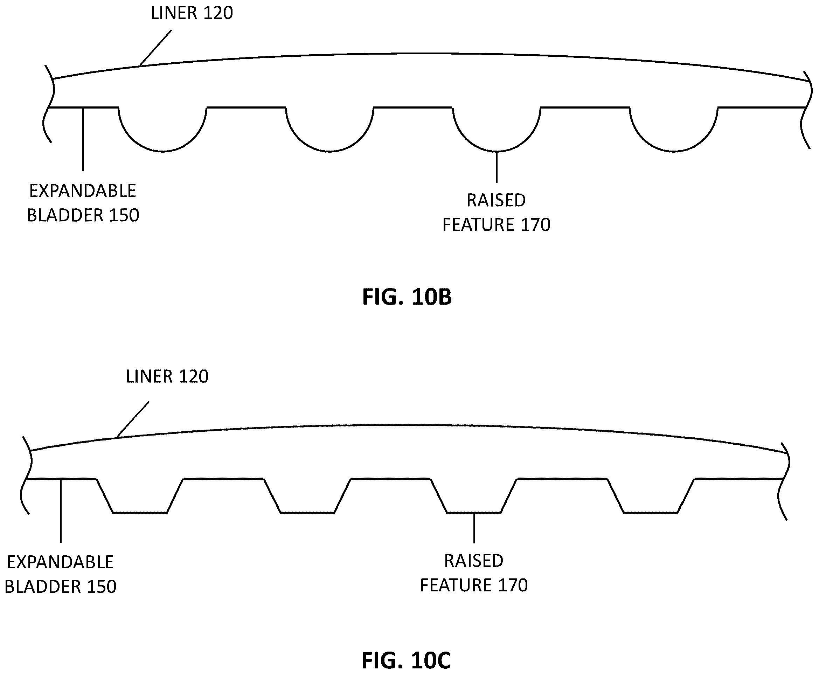

FIG. 10B is a cross section view of a portion of a liner 120 having a vasocompression element 130 with one or more raised features 170. In this illustrative embodiment, the raised features 170 have a rounded shape facing the patient who is wearing the head covering 100.

FIG. 10C is a cross section view of a portion of a liner 120 having a vasocompression element 130 with one or more raised features 170. In this illustrative embodiment, the raised features 170 have a rectangular pyramid shape with a flat top section facing the patient who is wearing the head covering 100.

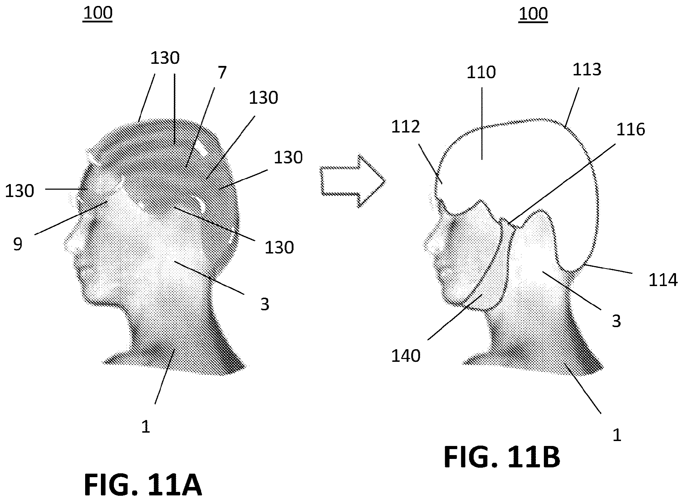

FIG. 11A is a left side view of a patient's head 3 who is wearing a vasocompression element used to deliver vasocompression in the blood supply of the patient while receiving chemotherapy. The outer shell 110 of the head covering 100 is removed in this view (but can be seen in the view of FIG. 11B). An array of different sized and shaped vasocompression elements 130 are visible in this view. Additionally or optionally, the one or more elements 130 may be configured as an inflatable liner which is connected to a pump or controller 180/190 (not visible in this view). In this configuration, the inflatable liner vasocompression elements 130 are configured to deliver vasocompression to the scalp 7 and eyebrows 9.

FIG. 11B is a left side view of the patient in FIG. 11A showing a complete head covering 100 with the shell 110 in place. The head covering 100 is held to the head 3 using an exemplary submandibular strap 140. FIG. 11B illustrates an exemplary position of the head covering 100 on the head 3 for inducing vasocompression in the blood supply of a patient 1 receiving chemotherapy using the vasocompression element configuration of FIG. 11A. In one aspect, the head covering 110 will deliver vasocompression for protection of the scalp 7 and eyebrows 9.

The head coverings 100 illustrated and described in FIGs. 9, 10, 11A and 11B are subject to a number of variations and alternatives. Turning now to a number of additional details of the features and operations of the various head covering 100 embodiments.

In one embodiment, there is provided a head covering 100 for inducing vasocompression in the blood supply of a patient receiving chemotherapy having a shell having a front edge, a back edge, a top, a left side edge and a right side edge. There is a liner within the shell shaped to at least partially accommodate the hair of the patient. There is also included at least one vasocompression element within the outer shell and a head covering retention system coupled to the shell.

In one aspect, the head covering retention system 140 may include a submandibular strap connected to the shell 110. Optionally, the head covering retention system 140 may include one or more of a rear strap, a front strap or a neck strap connected to the shell. In some embodiments, the shell retention system comprises one or more adjustment elements. Optionally, the head covering retention system 140 may include a hook and loop fastener, a buckle, a quick release mechanism or an adjustment feature.

In additional aspects, the at least one vasocompression element is an expandable bladder 150 within the liner 120. In an additional aspect, the at least one vasocompression element is coupled to the shell. There may also be a pressure source 160 in communication with the expandable bladder. Additionally or optionally there may be provided a continuous perimeter 160 connecting the front edge 112, the back edge 114, the left side edge 116 and the right side edge 118. Additionally, in one embodiment the head covering 100 is a continuous structure from the occiput area to the eyebrows or to the forehead. Still further, there is provided a structure or an inflatable structure to compress the eyebrows for a vasocompression effect on the eyebrows. It is to be appreciated that the pressure source may be used alone or as part of a control system as configured according to control system 180 and connector 185 in FIG. 9 or according to control system 190 as configured in FIG. 10A. In one embodiment, the pressure source 160 is in communication with the components of the head covering 100 using a suitable connector 185 as shown in FIG. 9. In another embodiment, the pressure source 160 is mounted on the head covering 100 as shown in the configuration of the control system 190 in FIG. 10A.

In additional embodiments, the at least one vasocompression element includes an expandable feature responsive to a pressure source 160 to expand away from the shell. There may also be provided an expandable layer coupled to the liner wherein the at least one vasocompression element is on the expandable layer. Additionally or optionally, the at least one vasocompression element includes one or more raised features. The liner within the shell shaped to at least partially accommodate the hair of the patient may also include one or more features on the liner to engage with the hair of the patient. Still further, the liner or the at least one vasocompression element may be smooth, textured, have irregular shapes of include an array of evenly distributed protrusions having straight or circular semi-rigid, flexible or inflatable structures.

In still other aspects, there is included a gas source in communication with the liner or the at least one vasocompression element for inflation of a portion of the liner or a portion of the at least one vasocompression element. This system may also include at least one relief valve to prevent over pressurization of an inflatable element of the head covering 100. In various embodiments, the gas source is a manual pump mechanism, a bulb, an electric pump, or pressurized tank, cartridge or reservoir. Optionally, the gas source may be worn or carried by the patient. Still further, the head covering 100 may include fittings or connectors for use with house gas, clean dry air or other building supplied gas source. Additionally or optionally, there may also be provided a control system, such as control system 180 or control system 190 configured for adjusting the flow of gas from the gas source to maintain a desired pressure within the head covering 100.

In still further embodiments, the control system 180 or the control system 190 may be a gas source and control system adapted and configured to include other additional capabilities such as these described herein. The desired pressure within the head covering 100 is sufficient to produce a vasocompression result while minimizing discomfort or side effects to the patient or harm to surrounding structures. There may also be included a cooling gas source in communication with the liner or the at least one vasocompression element wherein a portion of the liner or a portion of the at least one vasocompression element is adapted to provide cooling to an interior portion of the head covering 100. There may also be included one or more pressure sensors within an interior portion of the head covering 100. The one or more pressure sensors may be selected and provided appropriately to generate a signal of an indication of pressure to the control system 180/190. Still further, the one or more pressure sensors are adapted to indicate an amount of pressure applied to a patient undergoing chemotherapy when the patient is wearing the head covering 100. Additionally, the one or more sensors may be configured to measure skin perfusion (O2 sat) or Doppler blood flow monitor or infra-red pulse oximeter sensor. The sensors described herein may also be adapted and configured to determine blood flow rates in the vasculature receiving vasocompression to determine that the flow has stopped or slowed sufficiently so that to prevent flow of blood containing a chemotherapeutic agent. In one aspect, the detection of substantially reduced blood flow or stopped blood flow could also be used as a trigger within the control system 180/190 to increase the amount of vasocompression by an additional margin to increase confidence that the amount of vasocompression applied is sufficient. This additional margin of vasocompression may be included in a feedback loop performed by the control system 180/190 that measures amount of blood flow, increases vasocompression, measures blood flow to determine if slowed or stopped and then increasing vasocompression level as needed. Thereafter, an additional step of increasing vasocompression level after detection of stopped blood flow may be added as a safety margin to ensure temporary and reversible vasocompression induced vascular closure.

Additionally, the head covering 100 may be coupled to a timer and a control system 180/190 adapted to provide an automatic deflation protocol when the timer has elapsed or to cease operation of any active vasocompression element. In a variation, the head covering 100 may be coupled to a timer and a control system 180/190 adapted for programmed automatic inflation and deflation of the liner or the one or more vasoconstriction elements or the activation and deactivation of the one or more vasoconstriction elements. The system 180/190 may also include a display and Bluetooth, near field or other wireless or wired communication capabilities. Still further, the system may communicate status, programming, patient information or other aspects of vasocompression therapy or operations to a mobile phone, network, remote network or health information system. In still other aspects, the display may be positioned on the head covering 100 for providing information to a health care provider or the patient. Additionally or optionally, a display may be located on, in, within or integral to a portion of the shell 110. An embodiment of the display may be on a movable mount to allow viewing of the display by the patient using the head covering. Additionally or optionally, the head covering may be adapted with a suitable mount, connector or connectivity to allow a smart phone or other separate electronic device to be mounted to the head covering and configured for electronic communications, interactions and control of the components of the head covering. In additional aspects, the head covering 100 may also include ear buds, headphones, speakers or other audio components adjacent to the ears of the patient. In these embodiments, the system is also configured to provide noise cancellation, music, media or other entertainment to the patient while undergoing chemotherapy or using the head covering 100.

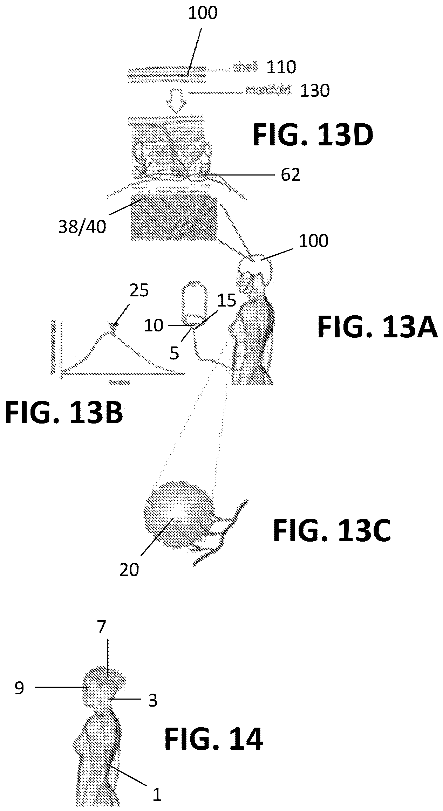

Additionally, with regard to the head covering 100 being operated by a timer sequence of an embodiment of the control system 180/190, the timing may be adapted to correspond to the expected half-life or toxicity level of the chemotherapy agent is use. In this way, the initiation of vasocompression therapy and the duration of the vasocompression therapy may be related to the introduction of the chemotherapeutic agent as well as the expected duration of the vasocompression therapy to correspond to the lingering toxicity in the blood of the residual chemotherapeutic agent. Considering the drug concentration profiles in FIGs. 3B and 13B, the duration of the vasocompression therapy would be selected to that time duration where the drug concentration level is lower than the peak level and approaching zero or pre-chemotherapy levels in the blood. This amount may vary by the patient as well as the specific toxicity profiles and half-lives of specific chemotherapeutic agents in use. As such, the timing function of the controller 180/190 may provide an automatic deflation protocol when the timer has elapsed which was selected to address the specifics of the patient and the agent used. Optionally, an additional timing margin may be added to provide additional confidence that the toxic level of chemotherapeutic agent in the blood poses no further thread or is a significantly reduced threat to the health of the scalp and hair structures (FIG. 8). Additionally or optionally, once the timer has elapsed or to cease operation of any active vasocompression element, the control system may stage or stagger the cessation of vasocompression therapy to sequence the re-introduction of blood flow after vasocompression therapy. In a variation, the head covering 100 may be coupled to a timer and a control system 180/190 adapted for programmed automatic inflation and deflation of the liner or the one or more vasoconstriction elements or the activation and deactivation of the one or more vasoconstriction elements in accordance with the guidelines discussed above.

In still other embodiments of a head covering 100 having a liner, the liner may be a rigid or semi-elastic layer configured for an exact fit for the patient to fit exactly the shape of the patient's head and hair.

The various hair covering embodiments described herein may also one or more selective pressure points positioned to compress one or more vessels of the cranial circulatory system of a patient. Additionally, other embodiments may include a vibrating element coupled to the liner or the at least one vasocompression element. In still further embodiments, the liner 120 and the at least one vasocompression element 130 are sized, shaped, or positioned relative to the head covering 100 based on the hair style of the patient.

In yet another alternative embodiment of a head covering 100, there is a cap having a front edge, a back edge, a top, a left side edge and a right side edge. There is a liner within the cap shaped to at least partially accommodate the hair of the patient. There is at least one vasocompression element within the cap. In one alternative, the cap comprises a flexible structure, a partially flexible structure, a combination of semi-rigid structure and flexible structure or an adjustable structure sized to remain in place on the head when the at least on vasocompression element is active.

In still other various alternative embodiments, the head covering 100 with the cap form factor may also be adapted and configured to include any of the above mentioned alternative, optional and additional variations and embodiments.

In some embodiments, one or more vasocompression elements are urged into contact with the scalp via springs, mechanical linkage, electromechanical drive, or electromagnetic arrangement.

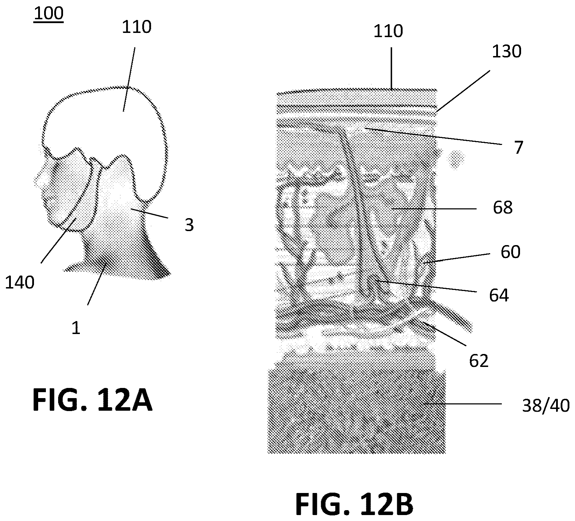

Turning now to FIGs. 12A-15 which will be used to describe an exemplary method of providing vasocompression to a patient undergoing chemotherapy. It is to be appreciated that the methods and variations thereof may be modified for implementation using any of the above described head covering 100 embodiments or vasocompression elements130.

FIG. 12A is a left side view of a patient's head who is wearing a head covering 100 including at least one vasocompression element 130 used to deliver vasocompression. This is the state of the patient prior to or shortly after initiation of the patient receiving chemotherapy. A submandibular strap 140 connected to a shell 110 of the head covering 100 is also visible in this view. The head covering 100 is configured to deliver vasocompression to the scalp 7 and eyebrows 9 of the patient.

FIG. 12B is a cross section of a portion of the hair, scalp and hair follicles of the patient in FIG. 12A. This view shows the hair 7 pressed against the scalp when the head covering 100 in donned (FIG. 12A) and prior to the delivery of vasocompression.

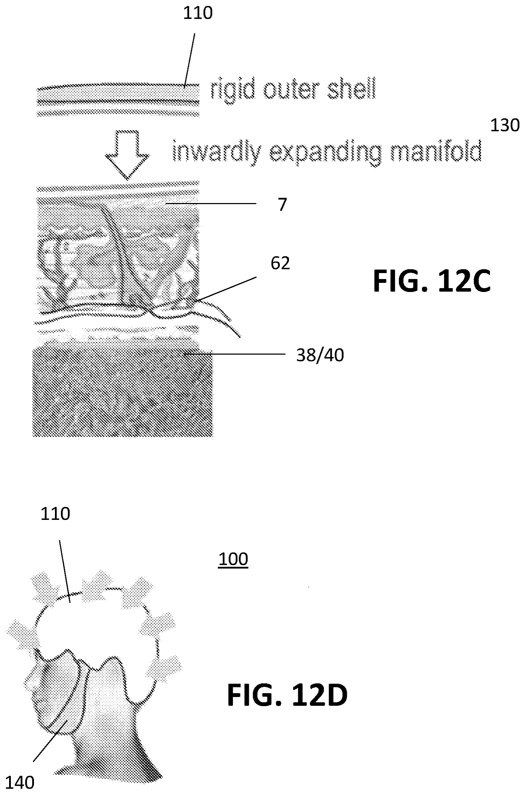

FIG. 12C is a cross section of a portion of the hair, scalp and hair follicles of the patient in FIG. 12B. This view shows the expansion of one or more vasocompression elements 130 in the head covering 100 (in the direction of the arrow) causing vasocompression within the vascular bed 62 of the follicle.

FIG. 12D is an exterior view of the patient 1 undergoing chemotherapy while vasocompression is being delivered by the head covering 100. The arrows indicating that vasocompression may be simultaneously or sequentially applied to numerous portions of the scalp 7 including the eyebrows 9.

FIG. 13A is a view of the patient of FIGs. 12A-12D receiving vasocompression from the head covering during a chemotherapy session which is nearly completed as indicated by the nearly empty IV bag 5 of chemotherapeutic agent 10.

FIG. 13B is the graph showing an expected concentration curve of the chemotherapeutic agent 10 in the patient's blood stream as a function of Drug Concentration (mg/L) and Time (min) for the status of the patient in FIG. 13A. The triangle 25 marks the estimated concentration based on the amount of chemotherapy agent delivered as indicated by the decreased level of fluid visible in the bag of FIG. 13A. The arrow indicates that the drug concentration amount is nearly at or just beyond the peak or maximum drug concentration.

FIG. 13C is a view of the advancing state of the process of chemotherapy-induced necrosis in the targeted tumor or malignant lesion of the patient of FIGs. 12A, 12D and 13A who has been receiving vasocompression during the delivery of the chemotherapeutic agent.

FIG. 13D is a cross section of the head covering 100, scalp and a hair follicle of the patient of FIGs. 12A, 12D and 13A who has been receiving vasocompression during the delivery of the chemotherapeutic agent 10 (FIG. 13B). This view shows that the vascular bed is completely free or substantially free of any chemotherapeutic agent 10 or that the level of chemotherapeutic agent 10 that reaches the vascular bed is maintained below a level to harm the follicles.

In contrast to the disastrous damage experiences by the unprotected patient in FIG. 6B, the patient who received vasocompression during chemotherapy has closed off vasculature 62 preventing the chemotherapeutic agent 10 from being delivered to or damaging the bulb 64 and associated hair shaft 70. As a result of the advantageous prevention of the introduction of the chemotherapeutic agent into the vascular bed of the scalp and eye brows, the patient who receives vasocompression as described herein in combination with chemotherapy avoids one or more of chemotherapy induced alopecia (CIA), chemotherapy drug-induced hair loss, hair follicle death and/or anagen effluvium.

FIG. 14 is a side view of the patient of FIG. 13A at a time period after the delivery of chemotherapy with vasocompression. The patient has maintained hair 7 on both the head 3 and the eyebrows 9 as a result of the beneficial effects of vasocompression.

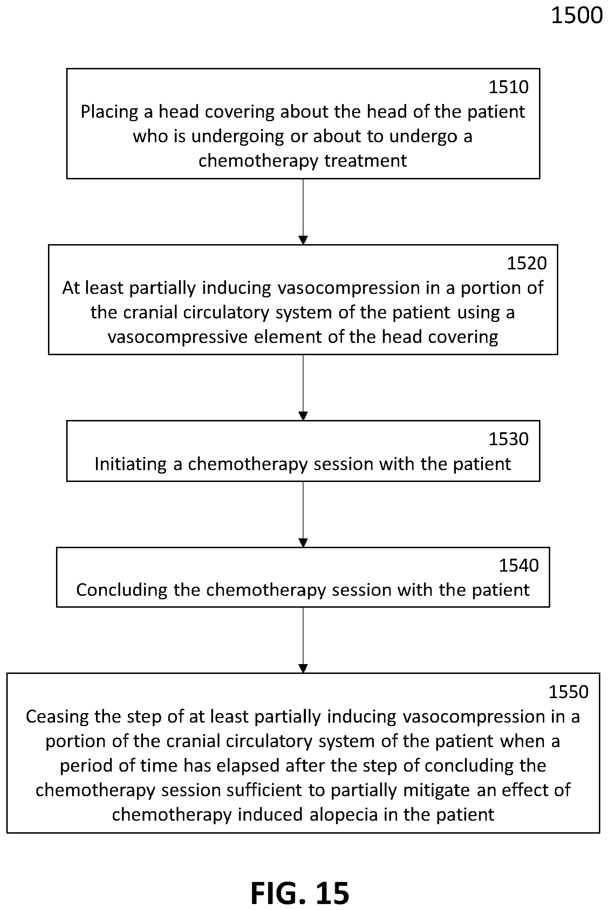

FIG. 15 is a flow chart of a representative method 1500 for providing vasocompression to a patient undergoing a chemotherapy treatment.

The representative method of inducing vasocompression in a cranial circulatory system of a patient receiving chemotherapy begins by placing a head covering 100 about the head of the patient (step 1510).

Next, there is a step of at least partially inducing vasocompression in a portion of the cranial circulatory system of the patient using a vasocompression element of the head covering 100 (step 1520).

Next, there is a step of initiating a chemotherapy session with the patient (step 1530).

Next, there is a step of concluding the chemotherapy session with the patient (step 1540).

Finally, there is a step of ceasing the step of at least partially inducing vasocompression in a portion of the cranial circulatory system of the patient when a period of time has elapsed after the step of concluding the chemotherapy session sufficient to partially mitigate an effect of chemotherapy induced alopecia in the patient (step 1550).

Optionally or additionally, the step of inducing vasocompression in a portion of the cranial circulatory system may be adjusted before, during or after the steps of initiating or concluding the chemotherapy session.

The method 1500 may also include engaging a head covering retention system 140 to immobilize the head covering 100 relative to the head of the patient.

The period of time that has elapsed after the step of concluding the chemotherapy session is more than 15 minutes, 30 minutes, 45 minutes, 1 hour, 2 hours, 3 hours or a period of time related to when the level of drug concentration in the patient's blood stream is below a level to harm the hair follicles.

In an additional aspect of the method, the vasocompression element 130 of the head covering 100 is a liner 120 wherein a step of expanding the liner or a step of contracting the liner is performed during the step of at least partially inducing vasocompression in a portion of the cranial circulatory system of the patient. Additionally or optionally, there is an increase or a decrease in the amount of vasocompression during the step of expanding the liner corresponding to an increase of the force of the liner against the scalp of the patient.

In an additional aspect of the method, there is provided a vasocompression element of the head covering that is a liner having one or more features wherein a step of expanding the liner or a step of contracting the liner is performed during the step of at least partially inducing vasocompression in a portion of the cranial circulatory system of the patient. In a still further aspect, there is an increase in the amount of vasocompression during the step of expanding the liner corresponding to an increase of the force of the one or more features on the liner against the scalp of the patient. Additionally or optionally, there a decrease in the amount of vasocompression during the step of contracting the liner corresponding to a decrease of the force of the one or more features on the liner against the scalp of the patient.

In yet another variation on the method, the vasocompression element 130 of the head covering is an inflatable bladder wherein the degree of vasocompression during the step of at least partially inducing vasocompression in a portion of the cranial circulatory system of the patient corresponds at least in part to the amount of pressure within the inflatable bladder.

In yet another variation on the method, the vasocompression element 130 of the head covering is an inflatable bladder wherein the degree of vasocompression during the step of at least partially inducing vasocompression in a portion of the cranial circulatory system of the patient is increased or decreased by raising or lowering the pressure in the inflatable bladder against the skull.

In yet another variation, the vasocompression element of the head covering is one or more features arranged on an interior surface of the head covering which are urged into contact with a portion of the patient's scalp having hair follicles during the step of at least partially inducing vasocompression in a portion of the cranial circulatory system of the patient.

Another variation includes a vasocompression element of the head covering is one or more features arranged on an interior surface of the head covering which correspond to an arrangement of the patient's hair, wherein the one of more features act on the arrangement of the patient's hair to further the performance of the step of least partially inducing vasocompression in a portion of the cranial circulatory system of the patient.

In still another variation of the method, during the at least partially inducing vasocompression step the vasocompression element of the head covering compresses a portion of the scalp containing a plurality of hair follicles against a portion of the cranium to at least partially vasocompress the blood supply to the plurality of hair follicles. Additionally or optionally, there is provided a step wherein the portion of the cranium is one or more of a superior temporal line, an inferior temporal line, a parietal bone, a squamous suture, a temporal bone, a lambdoid suture, an occipital bone, a mastoid process, a sphenoid, a portion in proximity to the supraorbital foramen, a frontal bone, a coronal suture

In another alternative version of the method, during the at least partially inducing vasocompression step, the vasocompression element of the head covering is positioned to at least partially vasocompress an artery or a vein of the cranial circulatory system of the patient by compressing the artery or the vein against a portion of the cranium. Still further, the artery or the vein of the cranial circulatory system is a branch of an external carotid artery or a branch of the internal carotid artery, the artery or the vein of the cranial circulatory system is an ophthalmic artery, the artery or the vein of the cranial circulatory system is a superficial temporal artery, the artery or the vein of the cranial circulatory system is a posterior auricular artery, the artery or the vein of the cranial circulatory system is an occipital artery, the artery or the vein of the cranial circulatory system is a supraorbital artery or a supratrochlear artery. Additionally, optionally or in combination, the portion of the cranium is one or more of a superior temporal line, an inferior temporal line, a parietal bone, a squamous suture, a temporal bone, a lambdoid suture, an occipital bone, a mastoid process, a sphenoid, a portion in proximity to the supraorbital foramen, a frontal bone, a coronal suture. It is to be appreciated that the size, shape and orientation in use of the shell 110 along with the size, shape, overall position and relative positions of a front edge 112, a back edge 114, a top 113, a left side edge 116 and a right side edge 118 of a shell 110 may be advantageously selected alone or in any combination with one or more vasocompression element embodiments described herein to selectively apply active and controllable vasocompression to one or more of the arteries or veins of the cranial circulatory system listed above. In one specific example, considering the scalp receives a rich arterial supply via the external carotid artery and the ophthalmic artery (a branch of the internal carotid), there is provided an embodiment of a vasocompression element or arrangement of elements that will compress the feeding vessels at a higher pressure (not like a torniquet but rather a direct point pressure) than the pressure otherwise applied to the regions of the scalp/hair or eyebrows.

In an additional variation, the method includes the step of at least partially inducing vasocompression in a portion of the cranial circulatory system results from compression of a portion of the scalp containing a blood vessel of the cranial circulatory system of the patient against a portion of the periosteum of the patient.

In an additional variation, the method includes the step of at least partially inducing vasocompression in a portion of the cranial circulatory system is produced by interaction between the vasocompression element of the head covering and a blood vessel of the cranial circulatory system of the patient.

In still another variation, there is a step of at least partially inducing vasocompression in a portion of the cranial circulatory system further comprising compressing a portion of the scalp against a portion of the periosteum to induce vasocompression in a portion of a vascular bed of a hair follicle.

In another alternative version of the method, there is a step wherein the vasocompression element of the head covering is positioned with respect to the patient's head wherein during the engaging step the vasocompression element is positioned in proximity a specific vein or a specific artery of the cranial circulatory system of the patient to at least partially vasocompress the specific vein or the specific artery.

Another alternative of the method includes delivery of a vasoconstrictor medication to the patient before, during or after any of the above mentioned methods. In one aspect, the vasoconstrictor medication is one of an alpha-adrenoceptor agonist, a vasopressin analog, an epinephrine, a norepinephrine, a phenylephrine, a dopamine, a dobutamine, a migraine medication, a headache medication, a serotonin 5-hydroxytryptamine agonist, and a triptans. Additionally, the above methods and devices and all variations thereof may be used to delivery vasocompression while a patient is receiving chemotherapy by an injection, an infusion, a targeted therapy, a hormone therapy or an immunotherapy. Still further, these methods and devices of providing vasocompression may be employed before or while the patient is receiving chemotherapy by an intravenous method, an intrathecal method, an intra-arterial method, an intracavitary method, an intramuscular method, an intralesional method, or an intravesical method. Additionally or optionally, because these methods and devices of providing vasocompression are compact, portable and mobile with the patient, the methods and devices may be readily employed regardless of whether the patient is receiving chemotherapy in a home, a health care provider office, a clinic, an outpatient infusion center, a hospital infusion center, or a hospital in-patient room.

Additional details of potentially suitable materials, details of construction of a head covering, a shell, a head covering retention feature or of one or more vasocompression elements may be appreciated by reference to US Patent 3,242,500 to Derr.

Additional details of potentially suitable materials, details of construction of a head covering, a shell, a head covering retention feature or of one or more vasocompression elements may be appreciated by reference to US Patent 9,743,701 to Javorek. By way of illustration and example, there is described herein additional aspects and alternatives for use with one or more head covering retention system embodiments with details related to, for example, a hook and loop fastener, a buckle, a quick release mechanism or an adjustment feature.

Additional details of potentially suitable materials, details of construction of one or more vasocompression elements may be appreciated by reference to US Design Patent 267,287 to Gooding.

Additional details of potentially suitable materials, details of construction of a head covering, a shell, a head covering retention feature or of one or more vasocompression elements may be appreciated by reference to US Patent Publication US 2006/0070170 to Copeland.

Additional details of potentially suitable complimentary compounds which may be used in combination with the devices and methods described herein as well as information related to the treatment of chemotherapy induced alopecia may be appreciated by reference to US Patent Publication US 2019/0240192 to Georgopoulos.

In addition to those listed above, still further additional alternative embodiments and combinations of the innovative features described herein are possible to deliver the active, personalized vasocompressive therapy described herein, such as for example:

Selective vasocompression of the key portions of the blood supply to the scalp - including programmed sequences of applying compression - for example to empty the capillary bed or block the main blood vessels of the scalp/skull.

In one aspect, a head covering is configured to include a set of separate vasocompression elements such as one along the periphery of the head covering or shell and other vasocompression elements within the interior of the shell. In one example, this arrangement would be a peripheral vasocompression zone and a central vasocompression zone. It is to be appreciated that these zones may be operated at different levels of pressure or amount of vasocompression by either manual control or via use of controller 180/190. These exemplary peripheral and central zones may be further divided, or additional other zones may be provided and incorporated into the head covering in order to more precisely deliver vasocompression therapy. Additionally or optionally, these zones may be operated at different pressures or levels of vasocompression as needed for the delivery of a pre-selected or patient specific vasocompression therapy profile.

A single device for protective vasocompression of all hair on the head - advantageously protecting both the hair on the scalp and eyebrows. Inclusion of eyebrows may be provided by additional vasocompressive elements positioned, sized and adapted for this purpose (SEE, e.g., FIGs. 10A and 11A).

A custom fit vasocompression device adapted and configured to patient specific head shape AND hair style. Additional embodiments include use of 3D imaging or scans of the patient head and hair styles, including hair styles to be used during chemotherapy sessions to ensure liner and shell fit for patient comfort and performance of the vasocompression therapy session.

Advanced active control and/or feedback vasocompression control system 180/190 with integrated pressure sensors and other detection devices within/integrated with the vasocompression device, including integration into the liner, expandable layer or shell, including for example stretch, strain or other measurement systems integrated into one or more components of the head covering 100 for inducing vasocompression in a patient.

The personalized vasocompression device embodiment may include one or more pressure sensors within an interior portion of a head covering. The one or more pressure sensors can provide an indication of pressure to the control system 180/190. The one or more pressure sensors can be adapted to indicate an amount of pressure applied to a patient undergoing chemotherapy when the patient is wearing the head covering. The head covering can further include one or more sensors to measure skin perfusion (O2 sat) or Doppler blood flow monitor or infra-red pulse oximeter sensor.

The inner liner (next to patient) can be a rigid or semi-elastic layer configured for an exact fit for the patient to fit exactly the shape of the patient's head and hair. The head covering retaining system can include a tightening system. The head covering can further include one or more selective pressure points positioned to compress one or more vessels of the cranial circulatory system of a patient. The head covering can further include a vibrating element coupled to the liner or the at least one vasocompression element. The liner and the at least one vasocompression elements can be sized, shaped, or positioned relative to the head covering based on the hair style of the patient.

The head covering can further include a gas source in communication with the liner or the at least one vasocompression element for inflation of a portion of the liner or a portion of the at least one vasocompression element. The head covering can further include at least one relief valve to prevent over pressurization of an inflatable element of the head covering. The gas source can be a manual pump mechanism, a bulb, an electric pump, or pressurized tank, cartridge or reservoir. The head covering can further include a control system 180/190 for adjusting the flow of gas from the gas source to maintain a desired pressure within the head covering. The desired pressure within the head covering can be sufficient to produce a vasocompression result while minimizing discomfort or side effects to the patient or harm to surrounding structures.

The head covering can further include a cooling gas source in communication with the liner or the at least one vasocompression element wherein a portion of the liner or a portion of the at least one vasocompression element is adapted to provide cooling to an interior portion of the head covering. Additionally, the gas used for inflation of a vasocompression element may also be cooled. The head covering can further include one or more pressure sensors within an interior portion of the head covering. The one or more pressure sensors can provide an indication of pressure to the control system 180/190. The one or more pressure sensors can be adapted to indicate an amount of pressure applied to a patient undergoing chemotherapy when the patient is wearing the head covering.

The personalized vasocompression system may also include advanced communication connectivity. As a result of this feature, a user may employ the personal vasocompression device with the functionality found on smart devices while undergoing therapy. Additionally, noise cancellation headphones or ear bud functionally may also be provided to enhance the user experience. The system may also include a display and Bluetooth, near field or other wireless or wired communication capabilities. Still further, the system may communicate status, programming, patient information or data, and/or any vasocompression device data related to all and any other aspects of vasocompression therapy or operations to an onboard storage device, a mobile phone, a communications network, remote network or health information system. In still other aspects, the display may be positioned on the head covering for providing information to a health care provider or the patient. In additional aspects, the head covering may also include ear buds, headphones, speakers or other audio components adjacent to the ears of the patient. In these embodiments, the system is also configured to provide noise cancellation, music, media or other entertainment to the patient while undergoing chemotherapy or using the head covering. Embodiments of the head covering may be externally powered by plugging into a suitable power source or may be powered wirelessly such as by use of an onboard battery system or an onboard rechargeable battery system.

Since it is the intention that hair is protected and preserved by the use of the various vasocompression devices and techniques, it is to be appreciated that aspects of the head covering 100 components are modified and adapted for the presence of patient hair 7. In additional or alternative embodiments, an additional liner or modified vasocompression element may include additional areas of compliance specific to be conformal with and accommodate elevated areas in the skull as well as those areas where the hair is collected or arranged during use of the head covering 100. In particular, the design and use of the head covering 100 in the delivery of vasocompression therapy factors in the circumstances that the patient hair, amount, style, placement in relation to the one or more vasocompression elements 130 will compress the hair (which is not compressible) and the hair then will compress the scalp thereby also contributing to the vasocompression of the vasculature.

Additionally or optionally, on or more surfaces of the one or more vasocompression elements may be modified with raised features of a range of different cross section shapes and tips or to have different length protrusions so as to accommodate different regions or points in the specific shape of the patient skull will be appropriately treated even in those regions where it is not on an even surface with the surrounding regions. Additionally, combinations of liner features, vasocompression element features as well as arrangement and placement of the patient's hair may also be used to accommodate patient specific cranial topography and shape to improve operation of the head covering as well as patient specific performance and comfort.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.
The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.

Examples of the disclosure include the following examples:
1. A head covering for inducing vasocompression in the blood supply of a patient receiving chemotherapy, comprising:
   A shell having a front edge, a back edge, a top, a left side edge and a right side edge;
   A liner within the shell shaped to at least partially accommodate the hair of the patient;
   At least one vasocompression element within the outer shell;
   A head covering retention system coupled to the shell.
2. The head covering of example 1 wherein the shell retention system comprises a submandibular strap connected to the shell.
3. The head covering of example 1 wherein the head covering retention system comprises one or more of a rear strap, a front strap or a neck strap connected to the shell.
4. The head covering of example 1 wherein the shell retention system comprises one or
   more
   adjustment elements.
5. The head covering of example 1 wherein the at least one vasocompression element is an expandable bladder within the liner.
6. The head covering of example 1 wherein the at least one vasocompression element is coupled to the shell.
7. The head covering of example 5 further comprising a pressure source in communication with the expandable bladder.
8. The head covering of example 1 further comprising a continuous perimeter connecting the front edge, the back edge, the left side edge and the right side edge.
9. The head covering of example 1 the at least one vasocompression element further comprising an expandable feature responsive to a pressure source to expand away from the shell.
10. The head covering of example 1 further comprising an expandable layer coupled to the liner wherein the at least one vasocompression element is on the expandable layer.
11. The head covering of example 1 the at least one vasocompression element further comprising one or more raised features.
12. The head covering of example 1 the liner within the shell shaped to at least partially accommodate the hair of the patient further comprising one or more features on the liner to engage with the hair of the patient.
13. The head covering of example 1 wherein the liner or the at least one vasocompression element are smooth.
14. The head covering of example 1 wherein the liner or the at least one vasocompression element comprise an array of evenly distributed protrusions having straight or circular inflatable structures.
15. The head covering of example 1 wherein the head covering retention system further comprising: a hook and loop fastener, a buckle, a quick release mechanism or an adjustment feature.
16. The head covering of example 1 further comprising a gas source in communication with the liner or the at least one vasocompression element for inflation of a portion of the liner or a portion of the at least one vasocompression element.
17. The head covering of example 16 further comprising at least one relief valve to prevent over pressurization of an inflatable element of the head covering.
18. The head covering of example 16 wherein the gas source is a manual pump mechanism, a bulb, an electric pump, or pressurized tank, cartridge or reservoir.
19. The head covering of example 16 further comprising a control system for adjusting the
   flow
   of gas from the gas source to maintain a desired pressure within the head covering.
20. The head covering of example 19 wherein the desired pressure within the head covering is sufficient to produce a vasocompression result while minimizing discomfort or side effects to the patient or harm to surrounding structures.
21. The head covering of example 1 further comprising a cooling gas source in communication with the liner or the at least one vasocompression element wherein a portion of the liner or a portion of the at least one vasocompression element is adapted to provide cooling to an interior portion of the head covering.
22. The head covering of example 1 further comprising one or more pressure sensors within an interior portion of the head covering.
23. The head covering of example 22 wherein the one or more pressure sensors provide an indication of pressure to the control system of claim 19.
24. The head covering of example 22 wherein the one or more pressure sensors are adapted to indicate an amount of pressure applied to a patient undergoing chemotherapy when the patient is
   wearing the head covering.
25. The head covering of example 1 further comprising one or more sensors to measure skin perfusion (02 sat) or Doppler blood flow monitor or infra-red pulse oximeter sensor or for confirmation that blood flow in vessels undergoing vasocompression has stopped or slowed sufficiently.
26. The head covering of example 1 further comprises a continuous structure from the occiput area to the eyebrows or to the forehead.
27. The head covering of any of examples 1-26 further comprising a structure or an inflatable structure to compress the eyebrows for a vasocompression effect on the eyebrows.
28. The head covering of example 1 further comprising a timer and a control system
   adapted to
   provide an automatic deflation protocol when the timer has elapsed or to cease operation of any active vasocompression element.
29. The head covering of example 1 further comprising a timer and a control system adapted for programmed automatic inflation and deflation of the liner or the one or more vasocompression elements or the activation and deactivation of the one or more vasocompression elements.
30. The head covering of example 1 wherein the liner is a rigid or semi-elastic layer configured for an exact fit for the patient to fit exactly the shape of the patient's head and hair.
31. The head covering of example 30 wherein the head covering retaining system comprises a tightening system.
32. The head covering of any of examples 1-31 further comprising one or more selective pressure points positioned to compress one or more vessels of the cranial circulatory system of a patient.
33. The head covering of any of examples 1-32 further comprising a vibrating element coupled to the liner or the at least one vasocompression element.
34. The head covering of any of examples 1-33 wherein the liner and the at least one vasocompression elements are sized, shaped, or positioned relative to the head covering based on the hair style of the patient.
35. A head covering for inducing vasocompression in the blood supply of a patient
   receiving
   chemotherapy, comprising:
   A cap having a front edge, a back edge, a top, a left side edge and a right side edge;
   A liner within the cap shaped to at least partially accommodate the hair of the patient; and At least one vasocompression element within the cap.
36. The head covering of example 35 wherein the cap comprises a flexible structure, a
   partially
   flexible structure, a combination of semi-rigid structure and flexible structure or an adjustable structure sized to remain in place on the head when the at least on vasocompression element is active.
37. The head covering of example 35 wherein the at least one vasocompression element is an expandable bladder within the cap or the liner.
38. The head covering of example 35 wherein the at least one vasocompression element is coupled to the cap or the liner.
39. The head covering of any of examples 35-38 further comprising a pressure source in communication with the expandable bladder or the at least one vasocompression element.
40. The head covering of example 35 further comprising a continuous perimeter
   connecting the
   front edge, the back edge, the left side edge and the right side edge.
41. The head covering of example 35 the at least one vasocompression element further comprising an expandable feature responsive to a pressure source to expand away from the cap.
42. The head covering of example 35 further comprising an expandable layer coupled to the liner wherein the at least one vasocompression element is on the expandable layer.
43. The head covering of example 35 the at least one vasocompression element further comprising one or more raised features.
44. The head covering of example 35 the liner within the shell shaped to at least partially accommodate the hair of the patient further comprising one or more features on the liner to engage with the hair of the patient.
45. The head covering of example 35 wherein the liner or the at least one vasocompression element are smooth.
46. The head covering of example 35 wherein the liner or the at least one vasocompression element comprise an array of evenly distributed protrusions having straight or circular inflatable
   structures.
47. The head covering of example 35 further comprising a gas source in communication with the liner or the at least one vasocompression element for inflation of a portion of the liner or a portion of the at least one vasocompression element.
48. The head covering of example 47 further comprising at least one relief valve to prevent
   over
   pressurization of an inflatable element of the head covering.
49. The head covering of example 47 wherein the gas source is a manual pump mechanism, a bulb, an electric pump, or pressurized tank, cartridge or reservoir.
50. The head covering of example 47 further comprising a control system for adjusting the flow of gas from the gas source to maintain a desired pressure within the head covering.
51. The head covering of example 50 wherein the desired pressure within the head covering is sufficient to produce a vasocompression result while minimizing discomfort or side effects to the patient or harm to surrounding structures.
52. The head covering of example 35 further comprising a cooling gas source in communication with the liner or the at least one vasocompression element wherein a portion of the liner or a
   portion of the at least one vasocompression element is adapted to provide cooling to an interior portion of the head covering.
53. The head covering of example 35 further comprising one or more pressure sensors within an interior portion of the head covering.
54. The head covering of example 53 wherein the one or more pressure sensors provide an indication of pressure to the control system of example 50.
55. The head covering of example 53 wherein the one or more pressure sensors are adapted to indicate an amount of pressure applied to a patient undergoing chemotherapy when the patient is wearing the head covering.
56. The head covering of example 35 further comprising one or more sensors to measure skin perfusion (02 sat) or Doppler blood flow monitor or infra-red pulse oximeter sensor.
57. The head covering of example 35 further comprises a continuous structure from the occiput area to the eyebrows or to the forehead.
58. The head covering of any of examples 35-57 further comprising a structure or an inflatable structure to compress the eyebrows for a vasocompression effect on the eyebrows.
59. The head covering of example 35 further comprising a timer and a control system adapted to provide an automatic deflation protocol when the timer has elapsed or to cease operation of any active vasocompression element.
60. The head covering of example 35 further comprising a timer and a control system adapted for programmed automatic inflation and deflation of the liner or the activation and deactivation of the one or more vasocompression elements.
61. The head covering of example 35 wherein the liner is a rigid or semi-elastic layer configured for an exact fit for the patient to fit exactly the shape of the patient's head and hair.
62. The head covering of example 61 wherein the head covering retaining system comprises a tightening system.
63. The head covering of any of examples 35-62 further comprising one or more selective pressure points positioned to compress one or more vessels of the cranial circulatory system of a patient.
64. The head covering of any of examples 35-63 further comprising a vibrating element coupled to the liner or the at least one vasocompression element.
65. The head covering of any of examples 35-64 wherein the liner and the at least one vasocompression elements are sized, shaped, or positioned relative to the head covering based on the hair style of the patient.
66. A method of inducing vasocompression in a cranial circulatory system of a patient receiving chemotherapy, comprising:
   Placing a head covering about the head of the patient;
   At least partially inducing vasocompression in a portion of the cranial circulatory system of the patient using a vasocompression element of the head covering;
   Initiating a chemotherapy session with the patient;
   Concluding the chemotherapy session with the patient;
      and
   Ceasing the step of at least partially inducing vasocompression in a portion of the cranial circulatory system of the patient when a period of time has elapsed after the step of
      concluding the chemotherapy session sufficient to partially mitigate an effect of chemotherapy induced alopecia in the patient.
67. The method of example 66 further comprising engaging a head covering retention system to immobilize the head covering relative to the head of the patient.
68. The method of example 66 wherein the period of time that has elapsed after the step of concluding the chemotherapy session up to 24 hours.
69. The method of example 66 wherein the period of time that has elapsed after the step of concluding the chemotherapy session is at least one hour.
70. The method of example 66 wherein the period of time that has elapsed after the step of concluding the chemotherapy session is at least 48 hours.
71. The method of example 66 wherein the vasocompression element of the head covering is a liner wherein a step of expanding the liner or a step of contracting the liner is performed during the step of at least partially inducing vasocompression in a portion of the cranial circulatory system of the patient.
72. The method of example 71 wherein there is an increase in the amount of vasocompression
   during the step of expanding the liner corresponding to an increase of the force of the liner against the scalp of the patient.
73. The method of example 71 wherein there is a decrease in the amount of vasoconstriction during the step of contracting the liner corresponding to a decrease of the force of the liner against the scalp of the patient.
74. The method of example 66 wherein the vasocompression element of the head covering is a liner having one or more features wherein a step of expanding the liner or a step of contracting the liner is performed during the step of at least partially inducing vasocompression in a portion of the cranial circulatory system of the patient.
75. The method of example 74 wherein there is an increase in the amount of vasocompression during the step of expanding the liner corresponding to an increase of the force of the one or more features on the liner against the scalp of the patient.
76. The method of example 74 wherein there is a decrease in the amount of
   vasocompression
   during the step of contracting the liner corresponding to a decrease of the force of the one or more features on the liner against the scalp of the patient.
77. The method of example 66 wherein the vasocompression element of the head covering is an inflatable bladder wherein the degree of vasocompression during the step of at least partially inducing vasocompression in a portion of the cranial circulatory system of the patient
   corresponds at least in part to the amount of pressure within the inflatable bladder.
78. The method of example 66 wherein the vasocompression element of the head covering is an inflatable bladder wherein the degree of vasocompression during the step of at least partially inducing vasocompression in a portion of the cranial circulatory system of the patient is
   increased or decreased by raising or lowering the pressure in the inflatable bladder against the skull.
79. The method of example 66 wherein the vasocompression element of the head covering is one or more features arranged on an interior surface of the head covering which are urged into contact with a portion of the patient's scalp having hair follicles during the step of at least partially inducing vasocompression in a portion of the cranial circulatory system of the patient.
80. The method of example 66 wherein the vasocompression element of the head covering is one or more features arranged on an interior surface of the head covering which correspond to an arrangement of the patient's hair, wherein the one of more features act on the arrangement of the patient's hair to further the performance of the step of least partially inducing vasocompression
   in a portion of the cranial circulatory system of the patient.
81. The method of example 66 wherein during the at least partially inducing vasocompression step the vasocompression element of the head covering compresses a portion of the scalp containing a plurality of hair follicles against a portion of the cranium to at least partially vasocompress the blood supply to the plurality of hair follicles.
82. The method of example 81 wherein the portion of the cranium is one or more of a superior
   temporal line, an inferior temporal line, a parietal bone, a squamous suture, a temporal bone, a lambdoid suture, an occipital bone, a mastoid process, a sphenoid, a portion in proximity to the supraorbital foramen, a frontal bone, a coronal suture.
83. The method of example 66 wherein during the at least partially inducing vasocompression step the vasocompression element of the head covering is positioned to at least partially
   vasocompress an artery or a vein of the cranial circulatory system of the patient by compressing the artery or the vein against a portion of the cranium.
84. The method of example 83 wherein the artery or the vein of the cranial circulatory system is a branch of an external carotid artery or a branch of the internal carotid artery.
85. The method of example 83 wherein the artery or the vein of the cranial circulatory
   system is
   an ophthalmic artery.
86. The method of example 83 wherein the artery or the vein of the cranial circulatory system is a superficial temporal artery.
87. The method of example 83 wherein the artery or the vein of the cranial circulatory system is a posterior auricular artery.
88. The method of example 83 wherein the artery or the vein of the cranial circulatory system is an occipital artery.
89. The method of example 83 wherein the artery or the vein of the cranial circulatory system is a supraorbital artery or a supratrochlear artery.
90. The method of example 83 wherein the portion of the cranium is one or more of a
   superior
   temporal line, an inferior temporal line, a parietal bone, a squamous suture, a temporal bone, a lambdoid suture, an occipital bone, a mastoid process, a sphenoid, a portion in proximity to the supraorbital foramen, a frontal bone, a coronal suture.
91. The method of example 66 wherein the step of at least partially inducing vasocompression in a portion of the cranial circulatory system results from compression of a portion of the scalp
   containing a blood vessel of the cranial circulatory system of the patient against a portion of the periosteum of the patient.
92. The method of example 66 wherein the step of at least partially inducing vasocompression in a portion of the cranial circulatory system is produced by interaction between the vasocompression element of the head covering and a blood vessel of the cranial circulatory system of the patient.
93. The method of example 66 the step of at least partially inducing vasocompression in a portion of the cranial circulatory system further comprising compressing a portion of the scalp against a portion of the periosteum to induce vasocompression in a portion of a vascular bed of a hair follicle.
94. The method of example 66 wherein the vasocompression element of the head covering is positioned with respect to the patient's head wherein during the engaging step the vasocompression element is positioned in proximity a specific vein or a specific artery of the cranial circulatory system of the patient to at least partially vasocompress the specific vein or the specific artery.
95. The method of example 94 wherein the specific vein or the specific artery is any of the blood vessels in any of examples 84-89.
96. The method of example 66 further comprising delivery of a vasoconstrictor medication to the patient before, during or after any step of example 66.
97. The method of example 94 wherein the vasoconstrictor medication is one of an alpha- adrenoceptor agonist, a vasopressin analog, an epinephrine, a norepinephrine, a phenylephrine, a
   dopamine, a dobutamine, a migraine medication, a headache medication, a serotonin 5- hydroxytryptamine agonist, and a triptans.
98. The method or device as in any of the above claims wherein the patient is receiving chemotherapy by an injection, an infusion, a targeted therapy, a hormone therapy or an immunotherapy.
99. The method or device of example 98 wherein the patient is receiving chemotherapy by an intravenous method, an intrathecal method, an intra-arterial method, an intracavitary method, an intramuscular method, an intralesional method, or an intravesical method.
100. The method or device of examples 98 and 99 wherein the patient is receiving chemotherapy in a home, a health care provider office, a clinic, an outpatient infusion center, a hospital infusion
   center, or a hospital in-patient room.
101. The method of any of examples 66-100 wherein the method is practiced by a device as in any of examples 1-65.

## Claims

1. A system for inducing vasocompression in a cranial circulatory system of a patient receiving chemotherapy, comprising:
a head covering (100) for placing about the head of the patient;
at least one vasocompression element (130) of the head covering (100) for at least partially inducing vasocompression in a portion of the cranial circulatory system of the patient;
a controller (180, 190) configured to execute the vasocompression to the patient using the at least one vasocompression element (130) of the head covering (100);
and wherein the controller (180, 190) is configured to cease inducing the vasocompression when a period of time has elapsed after a chemotherapy session.

2. The system of claim 1 wherein the vasocompression element (130) of the head covering (100) is an inflatable bladder wherein the degree of vasocompression during the step of at least partially inducing vasocompression in a portion of the cranial circulatory system of the patient corresponds at least in part to the amount of pressure within the inflatable bladder.

3. The system of claim 1 wherein the vasocompression element (130) of the head covering (100) is an inflatable bladder wherein the degree of vasocompression during the step of at least partially inducing vasocompression in a portion of the cranial circulatory system of the patient is increased or decreased by raising or lowering the pressure in the inflatable bladder against the skull.

4. The system of claim 1 wherein the vasocompression element (130) of the head covering(100) is one or more features arranged on an interior surface of the head covering which are urged into contact with a portion of the patient's scalp having hair follicles during the step of at least partially inducing vasocompression in a portion of the cranial circulatory system of the patient.

5. The system of claim 1 wherein the vasocompression element (130) of the head covering(100) is one or more features arranged on an interior surface of the head covering which correspond to an arrangement of the patient's hair, wherein the one of more features act on the arrangement of the patient's hair to further the performance of the step of least partially inducing vasocompression in a portion of the cranial circulatory system of the patient.

6. The system of claim 1 wherein the at leat one vasocompression element (130) of the head covering (100) are configured to compresses a portion of the scalp containing a plurality of hair follicles against a portion of the cranium to at least partially vasocompress the blood supply to the plurality of hair follicles.

7. The system of claim 6 wherein the portion of the cranium is one or more of a superior temporal line, an inferior temporal line, a parietal bone, a squamous suture, a temporal bone, a lambdoid suture, an occipital bone, a mastoid process, a sphenoid, a portion in proximity to the supraorbital foramen, a frontal bone, a coronal suture.

8. The system of claim 1 wherein the at least one vasocompression element (130) of the head covering (100) is configured to be positioned to at least partially vasocompress an artery or a vein of the cranial circulatory system of the patient by compressing the artery or the vein against a portion of the cranium.

9. The system of claim 8 wherein the artery or the vein of the cranial circulatory system is a branch of an external carotid artery or a branch of the internal carotid artery,an ophthalmic artery, a superficial temporal artery, a posterior auricular artery, an occipital artery, a supraorbital artery or a supratrochlear artery; or . wherein the portion of the cranium is one or more of a superior temporal line, an inferior temporal line, a parietal bone, a squamous suture, a temporal bone, a lambdoid suture, an occipital bone, a mastoid process, a sphenoid, a portion in proximity to the supraorbital foramen, a frontal bone, a coronal suture.

10. The system of claim 1 wherein least partially inducing vasocompression in a portion of the cranial circulatory system results from compression of a portion of the scalp containing a blood vessel of the cranial circulatory system of the patient against a portion of the periosteum of the patient; or
wherein the at least partially inducing vasocompression in a portion of the cranial circulatory system is produced by interaction between the vasocompression element (130) of the head covering and a blood vessel of the cranial circulatory system of the patient; or
wherein the at least partially inducing vasocompression in a portion of the cranial circulatory system further comprising compressing a portion of the scalp against a portion of the periosteum to induce vasocompression in a portion of a vascular bed of a hair follicle; or
wherein the vasocompression element of the head covering is configured to be positioned with respect to the patient's head wherein during the engaging step the vasocompression element (130) is positioned in proximity a specific vein or a specific artery of the cranial circulatory system of the patient to at least partially vasocompress the specific vein or the specific artery.

11. The system of claim 1, wherein the control system includes a pressure source, such as in communication with an expandable bladder or the at least one vasocompression element (130).

12. The system of claim 1, comprising a gas source in communication with the at least one vasocompression element for inflation of a portion of the at least one vasocompression element (130), wherein the gas source for instance is a manual pump mechanism, a bulb, an electric pump, or pressurized tank, cartridge or reservoir.

13. The system of claim 12, wherein the controller is configured to adjust the flow of gas from the gas source to maintain a desired pressure within the head covering, wherein the desired pressure within the head covering is preferably sufficient to produce a vasocompression result while minimizing discomfort or side effects to the patient or harm to surrounding structures.

14. The system of claim 1, further comprising one or more pressure sensors within an interior portion of the head covering(100), wherein the one or more pressure sensors preferably provide an indication of pressure to the controller of claim 13, and/or wherein the one or more pressure sensors are preferably adapted to indicate an amount of pressure applied to a patient undergoing chemotherapy when the patient is wearing the head covering (100).

15. The system of claim 1, further comprising one or more sensors to measure skin perfusion (O2 sat) or Doppler blood flow monitor or infra-red pulse oximeter sensor; or further comprising a continuous structure from the occiput area to the eyebrows or to the forehead; or further comprising a structure or an inflatable structure to compress the eyebrows for a vasocompression effect on the eyebrows.
